(19) Europäisches Patentamt
European Patent Office
Office européen des brevets

(11) Veröffentlichungsnummer: **0 006 502**
**B1**

(12) # EUROPÄISCHE PATENTSCHRIFT

(45) Veröffentlichungstag der Patentschrift: **13.01.82**

(51) Int. Cl.³: **C 07 C 143/86**

(21) Anmeldenummer: **79101818.7**

(22) Anmeldetag: **08.06.79**

(54) **Verfahren zur Herstellung von O-substituierten N-Hydroxysulfamidsäurehalogeniden.**

(30) Priorität: **24.06.78 DE 2827871**

(43) Veröffentlichungstag der Anmeldung:
**09.01.80 Patentblatt 80/1**

(45) Bekanntmachung des Hinweises auf die Patenterteilung:
**13.01.82 Patentblatt 82/2**

(84) Benannte Vertragsstaaten:
**BE CH DE FR GB IT**

(56) Entgegenhaltungen:
**DE - A - 2 164 176**
**FR - A - 1 263 547**

(73) Patentinhaber: **BASF Aktiengesellschaft**
**Carl-Bosch-Strasse 38**
**D-6700 Ludwigshafen (DE)**

(72) Erfinder: **Parg, Adolf, Dr.**
**Paray-Le-Monial-Strasse 8**
**D-6702 Bad Duerkheim 5 (DE)**
Erfinder: **Hamprecht, Gerhard, Dr.**
**Rote-Turm-Strasse 28**
**D-6940 Weinheim (DE)**

**EP 0 006 502 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Verfahren zu herstellung von O-substituierten N-Hydroxysulfamidsäurehalogeniden

Die Erfindung betrifft neue O-substituierte N-Hydroxysulfamidsäurehalogenide und ein Verfahren zu ihrer Herstellung durch Umsetzung von Sulfamidsäuren mit Säurehalogeniden in bestimmten Molverhältnissen.

Es ist aus der deutschen Offenlegungsschrift 2 164 176 bekannt, daß man N-Alkyl- bzw. N-Cycloalkyl-sulfamidsäurehalogenide durch Umsetzung von Sulfamidsäuren mit einem Säurehalogenid der schwefligen Säure, Phosphorsäure, phosphorigen Säure, Kohlensäure oder Oxalsäure herstellt. Thionylchlorid, Thionylbromid, Phosphorpentachlorid, Phosphortrichlorid, Phosphoroxychlorid, Phosphorpentabromid, Phosphortribromid, Phosgen, Oxalsäurechlorid und Oxalsäurebromid werden als bevorzugte Säurehalogenide geoffenbart. Wie die Ausführungsbeispiele zeigen, wird jeweils nur ein einziges Säurehalogenid verwendet. Außer Phosgen und Phosphorpentachlorid in je einem Beispiel, wird nur Thionylchlorid als Ausgangshalogenid in den übrigen Beispielen veranschaulicht. Die deutsche Offenlegungsschrift lehrt ausdrücklich, daß mindestens die stöchiometrische Menge an Säurehalogenid, vorzugsweise aber ein Verhältnis von 1,1 bis 2 Mol Säurehalogenid je Mol Ausgangsstoff II für die Reaktion notwendig ist, und diese Umsetzung nur mit am Stickstoffatom monosubstituierten Sulfamidsäurehalogeniden, deren Substituenten über ein Kohlenstoffatom substituierende aliphatische und cycloaliphatische Reste sind, durchgeführt wird.

Es ist aus Houben-Weyl, Methoden der Organischen Chemie, Band VI/3, Seite 123, bekannt, daß Äther mit Sulfurylchlorid bervorzugt unter Chlorierung am $\alpha$-Kohlenstoffatom reagieren; durch HCl-Abspaltung mit $\beta$-ständigem Wasserstoff entstehen über die Vinyläther auch höherchlorierte Produkte, z.B aus dem Diäthyläther der Äthyl-(1,2-dichloräthyl)-äther, O-substituierte organische Hydroxylamine ergeben mit Thionylchlorid Sulfoxidoverbindungen (loc. cit., Band X/1, Seite 1 263):

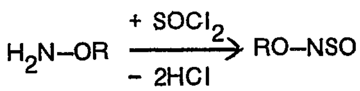


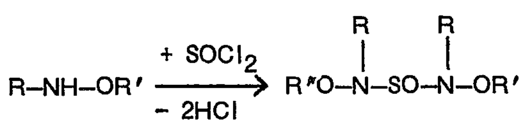


Mit Phosgen (loc., cit., Band X/1, Seite 1 262) reagieren O-Hydroxylamine bei 20°C zu N-Chlorcarbonyl-O-hydroxylaminen, bei erhöhter Temperatur (120 bis 125°C) hauptsächlich zu in 1,3,5-Stellung verätherten 1,3,5-Hydroxy-2,4,6-trioxohexahydro-1,3,5-triazinen.

Es wurde nun gefunden, daß man O-substituierte N-Hydroxy-sulfamidsäurehalogenide der Formel

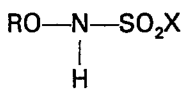
I,

in der R einen aliphatischen oder cycloaliphatischen Rest bedeutet und X ein Halogenatom bezeichnet, durch Umsetzung von Sulfamidsäuren mit einem Säurehalogenid vorteilhafterhält, wenn man Sulfamidsäuren der Formel

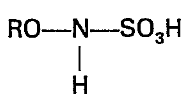
II,

in der R die vorgenannte Bedeutung hat, oder ihre sulfamidsauren Metallsalze mit einem Säurehalogenid der schwefligen Säure, Phosphorsäure, phosphorigen Säure, Kohlensäure oder Oxalsäure in einer Menge von 0,5 bis 1 Mol Säurehalogenid, bezogen auf 1 Mol Ausgangsstoff II, umsetzt.

Weiterhin wurden die neuen O-substituierten N-Hydroxysulfamidsäurehalogenide der Formel

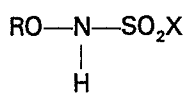
I,

in der R einen aliphatischen oder cycloaliphatischen Rest bedeutet und X ein Halogenatom bezeichnet, gefunden. Bevorzugt sind die neuen Sulfamidsäurechloride der Formel

$$RONHSO_2CL \qquad I,$$

in der R einen Alkylrest von 1 bis 7 Kohlenstoffatomen bedeutet, insbesondere O-Methyl-N-hydroxysulfamidsäurechlorid, O-Äthyl-N-hydroxysulfamidsäurechlorid, O-n-Heptyl-N-hydroxy-sulfamidsäurechlorid.

Die Umsetzung läßt sich für den Fall der Verwendung des Natriumsalzes der O-Äthyl-N-hydroxysulfamidsäure durch folgende Formeln wiedergeben:

$$2C_2H_5O{-}NHSO_3Na + PCl_5 \xrightarrow{-(PO_2Cl)} 2C_2H_5O{-}NHSO_2Cl + 2NaCl.$$

Im Hinblick auf den Stand der Technik liefert das erfindungsgemäße Verfahren auf einfache und wirtschaftliche Weise die neuen O-substituierten N-Hydroxysulfamidsäurehalogenide in guter Ausbeute und Reinheit. Die Reaktionszeit ist kurz, die Aufarbeitung des Reaktionsgemisches, gerade auch im Hinblick auf den Umweltschutz, einfach und betriebssicher. Im Hinblick auf den Stand der Technik sind alle diese vorteilhaften Ergebnisse überraschend.

Die Ausgangsstoffe II können beispielsweise nach einem Verfahren von W. Traube, H. Ohlendorf und H. Zander (Berichte der deutschen Chemischen Gesellschaft, Band 53 (1920), Seiten 1 477—1 492) in Form der Alkalisalze der O-Alkyl-N-hydroxysulfamidsäure hergestellt werden. Auch können sie in Analogie zu dem im J. Am. Chem. Soc., Band 73 (1951), Seite 5 507 beschriebenen Verfahren für n-Alkylsulfamate durch Umsetzung von O-substituierten Hydroxylaminen mit Schwefeltrioxid oder Amidosulfonsäure hergestellt werden.

Bevorzugte Ausgangsstoffe II und dementsprechend bevorzugte Endstoffe I sind solche, in deren Formeln R einen geradkettigen oder verzweigten Alkylrest mit 1 bis 20, insbesondere 1 bis 8 Kohlenstoffatomen, einen durch mehrere Alkoxygruppen, vorzugsweise 3 oder 2 Alkoxygruppen und insbesondere eine Alkoxygruppe mit 1 bis 7, insbesondere 1 bis 3 Kohlenstoffatomen substituierten Alkylrest mit 2 bis 20, insbesondere 2 bis 8, vorteilhaft 2 bis 6 Kohlenstoffatomen, oder einen Cycloalkylrest mit 4 bis 8 Kohlenstoffatomen bedeutet, und X ein Fluoratom oder insbesondere Chloratom bezeichnet. Die genannten Reste können noch durch unter den Reaktionsbedingungen inerte Gruppen und/oder Atome, z.B. Chloratome, Bromatome, Alkylgruppen, Alkoxygruppen mit jeweils 1 bis 4 Kohlenstoffatomen, Carbalkoxygruppen mit 2 bis 4 Kohlenstoffatomen, Cycloalkylgruppen mit 4 bis 6 Kohlenstoffatomen, substituiert sein. Bevorzugte Ausgangsstoffe II und Endstoffe I sind insbesondere solche, in deren Formel R einen Methyl-, Äthyl-, n-Propyl-, iso-Propyl-, n-Butyl-, iso-Butyl-, sek.-Butyl-, n-Pentyl-, n-Hexyl- oder n-Heptylrest bezeichnet.

Beispielsweise kommen folgende Sulfamidsäuren II in Betracht:O-Methyl-N-hydroxysulfamidsäure, O-Äthyl-N-hydroxysulfamidsäure, O-n-Propyl-N-hydroxysulfamidsäure, O-Isopropyl-N-hydroxysulfamidsäure, O-n-Butyl-N-hydroxysulfamidsäure, O-Isobutyl-N-hydroxysulfamidsäure, O-sek.-Butyl-N-hydroxysulfamidsäure, O-(1-Äthyl)-n-propyl-N-hydroxysulfamidsäure, O-(1,2-Dimethyl)-n-propyl-N-hydroxysulfamidsäure, O-n-Pentyl-N-hydroxysulfamidsäure, O-Cyclopentyl-N-hydroxysulfamidsäure, O-n-Hexyl-N-hydroxysulfamidsäure, O-Hexyl-(3)-N-hydroxysulfamidsäure, O-Cyclohexyl-N-hydroxysulfamidsäure, O-Heptyl-(4)-N-hydroxysulfamidsäure, O-(2-Methyl-1-äthyl)-n-propyl-N-hydroxysulfamidsäure, O-(1,2,2-Trimethyl)-n-propyl-N-hydroxysulfamidsäure, O-(1,3-Dimethyl)-n-butyl-N-hydroxysulfamidsäure, O-(1,2-Dimethyl)-n-butyl-N-hydroxysulfamidsäure, O-(1,2-Dimethyl)-n-hexyl-N-hydroxysulfamidsäure, O-(1-Cyclohexyl)-äthyl-N-hydroxysulfamidsäure, O-2-Chlorisopropyl-N-hydroxysulfamidsäure, O-2-Chlorpropyl-N-hydroxysulfamidsäure, O-3-Chlorpropyl-N-hydroxysulfamidsäure, O-3-Brompropyl-N-hydroxysulfamidsäure, O-(1-Chlormethyl)-n-propyl-N-hydroxysulfamidsäure; O-tert.-Butyl-, O-Pentyl-(2)-, O-n-Heptyl-, O-n-Octyl-, O-n-Nonyl-, O-n-Decyl-, O-2-Äthylhexyl-, O-2-Äthylpentyl-, O-3-Äthylpentyl-, O-2,3-Dimethyl-n-butyl-, O-2-Methylpentyl-, O-3-Methyl-pentyl-, O-2-Methylheptyl-, O-3-Methylheptyl-, O-4-Methylheptyl-, O-3-Äthylhexyl-, O-2,3-Dimethylhexyl-, O-2,4-Dimethylhexyl-, O-2,5-Dimethylhexyl-, O-Undecyl-, O-Dodecyl-, O-Tridecyl-, O-Tetradecyl-, O-Pentadecyl-, O-Hexadecyl-, O-Heptadecyl-, O-Octadecyl-, O-Nonadecyl-, O-Eicosyl-N-hydroxysulfamidsäure; die $\omega$-Methoxy-, $\omega$-Äthoxy-, $\omega$-n-Propoxy-, $\omega$-Isopropoxy-, $\omega$-n-Butoxy-, $\omega$-Isobutoxy-, $\omega$-sek.-Butoxy-, -tert.Butoxy-, -Pentoxy-, -Pentoxy-(2)-, -Pentoxy-(3)-, -n-Hexoxy-, -n-Heptoxy-verbindung der Äthyl-, n-Propyl-, Isopropyl-, n-Butyl-, Isobutyl-, sek.Butyl-, tert.-Butyl-, Pentyl-, Pentyl-(2)-, Pentyl-(3)-, n-Hexyl-, n-Heptyl-, n-Octyl-, n-Nonyl-, n-Decyl-, 2-Äthylhexyl-, 2-Äthylpentyl-, 3-Äthylpentyl-, 2,3-Dimethyl-n-butyl-, 2-Methylpentyl-, 3-Methylpentyl-, 2-Methylheptyl-, 3-Methylheptyl-, 4-Methylheptyl-, 3-Äthylhexyl-, 2,3-Dimethylhexyl-, 2,4-Dimethylhexyl-, 2,5-Dimethylhexyl-, Undecyl-, Dodecyl-, Tridecyl-, Tetradecyl-, Pentadecyl-, Hexadecyl-, Heptadecyl-, Octadecyl-, Nonadecyl-, Eicosyl-(O)-N-hydroxysulfamidsäure; entsprechende Methyl-, Äthyl-, n-Propyl-, Isopropyl-, n-Butyl-, Isobutyl, sek.-Butyl-, tert.-Butyl-, Pentyl-, Pentyl-(2)-, Pentyl-(3)-, n-Hexyl-, n-Heptyl-äther in 1-Stellung oder 2-Stellung der n-Propyl-, Isopropyl-, n-Butyl-, Isobutyl-, sel.-Butyl-, tert.-Butyl-, Pentyl-, Pentyl-(2)-, Pentyl-(3)-, n-Hexyl-, n-Heptyl-, n-Octyl-, n-Nonyl-, n-Decyl-, 2-Äthylhexyl-, 2-Äthylpentyl-, 3-Äthylpentyl-, 2,3-Dimethyl-n-butyl, 2-Methylpentyl-, 3-Methylpentyl-, 2-Methylheptyl-, 3-Methylheptyl-, 4-Methylheptyl-, 3-Äthylhexyl-, 2,3-Dimethylhexyl-, 2,4-Dimethylhexyl-, 2,5-Dimethylhexyl-, Undecyl, Dodecyl-, Tridecyl-, Tetradecyl-, Pentadecyl-, Hexadecyl-, Octadecyl-, Nonadecyl-, Eicosyl-(O)-N-hydroxysulfamidsäure bzw. 1-Stellung der Äthyl-(O)-N-hydroxysulfamidsäure.

Die Ausgangsstoffe II können in Gestalt von Sulfamidsäuren oder ihrer sulfamidsauren Metallsalze angewendet werden. Bevorzugte Metallsalze sind Alkali- oder Erdalkalisalze wie sulfamidsaures Magnesium, Calcium, Lithium, Kalium und insbesondere Natrium.

Die Ausgangsstoffe II werden mit dem Säurehalogenid in einem Verhältnis von 0,5 bis 1, vorzugsweise von 0,8 bis 1 Mol Säurehalogenid je Mol Ausgangsstoff II umgesetzt. Bevorzugte Säurehalogenide sind solche der allgemeinen Formel

$$Z—X \qquad III,$$

in der X ein Chlor- oder Fluoratom bedeutet und Z den Acylrest der vorgenannten Säuren bezeichnet, vorteilhaft Thionylchlorid, Thionylfluorid, Schwefeltetrafluorid, Phosgen, Oxalsäurechlorid und insbesondere Phosphorpentachlorid, Phosphortrichlorid und Phosphoroxychlorid.

Das verwendete Säurehalogenid oder ein Gemisch von Säurehalogeniden, insbesondere ein Gemisch mit Phosphoroxyhalogenid als einer Komponente des Gemischs, können bei dem erfindungsgemäßen Verfahren als Lösungsmittel dienen. Man führt die Reaktion aber zweckmäßig in einem unter den Reaktionsbedingungen inerten, organischen Lösungsmittel durch. Als unter den Reaktionsbedingungen inerte Lösungsmittel kommen in Frage: Halogenkohlenwasserstoffe, insbesondere Chlorkohlenwasserstoff z.B. Tetrachloräthylen, 1,1,2,2- und 1,1,1,2-Tetrachloräthan, Amylchlorid, Cyclohexylchlorid, Dichlorpropan, Methylenchlorid, Dichlorbutan, Isopropylbromit, n-Propylbromid, Butylbromid, Chloroform, Äthyljodid, Propyljodid, Chlornaphthalin, Dichlornaphthalin, Tetrachlorkohlenstoff, 1,1,1- und 1,1,2-Trichloräthan, Trichloräthylen, Pentachloräthan, 1,2-cis-Dichloräthylen, 1,2-Dichloräthan, 1,1-Dichloräthan, n-Propylchlorid, n-Butylchlorid, 2-, 3- und iso-Butylchlorid, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol, 1,10-Dibromdekan, 1,4-Dibrombutan; aliphatische oder cycloaliphatische Äther, z.B. n-Butyläthyläther, Di-n-butyläther, Di-iso-amyläther, Diisopropyläther, Cyclohexylmethyläther, Diäthyläther, Tetrahydrofuran, Dioxan, $\beta,\beta'$-Dichlordiäthyläther; Nitrile wie Acetonitril, Propionitril, Butyronitril, Isobutyronitril, Benzonitril, o-, m-, p-Chlorbenzonitril; aliphatische oder cycloaliphatische Kohlenwasserstoffe, z.B. Pentan, Hexan, Heptan, Nonan, $\alpha$-Pinen, o-, m-, p-Cymol, Benzinfraktionen, insbesondere mit einem Siedepunkt von 70 bis 190°C, Cyclohexan, Methylcyclohexan, Petroläther, Dekalin, Ligroin, 2,2,4-Trimethylpentan, 2,2,3-Trimethylpentan, 2,3,3,-Trimethylpentan, Octan; aromatische Kohlenwasserstoffe, z.B. Benzol, Toluol, Äthylbenzol, o-, m-, p-Xylol, Mesitylen; und entsprechende Gemische. Vorteilhaft ist ein Mengenverhältnis von 20 bis 1 000 Gewichtsprozent, vorzugsweise von 35 bis 300, insbesondere von 65 bis 150 Gewichtsprozent Lösungsmittel, bezogen auf Ausgangsstoff II.

Die Reaktion wird in der Regel bei einer Temperatur von −10 bis +130°C, vorzugsweise 10 bis 120°C, insbesondere von 50 bis 100°C, drucklos oder unter Druck, kontinuierlich oder diskontinuierlich durchgeführt.

Die Reaktion kann wie folgt durchgeführt werden: Ein Gemisch von Ausgangsstoff II, Säurehalogenid und Lösungsmittel wird während 2 bis 8 Stunden bei der Reaktionstemperatur gehalten. Man kann das Säurechlorid oder den Ausgangsstoff II zusammen mit Lösungsmittel vorlegen und dann die andere Komponente zugeben. Aus dem Reaktionsgemisch wird der Endstoff I in üblicher Weise, z.B. durch fraktionierte Destillation, abgetrennt. In einer vorteilhaften Ausführungsform vermischt man beispielsweise eine Suspension der Sulfamidsäure II oder ihrer Salze mit einem der vorgenannten Halogenkohlenwasserstoffe, zweckmäßig in einer Menge von 65 bis 300 Gewichtsprozent der vorgenannten Halogenkohlenwasserstoffe, bezogen auf Ausgangsstoff II, z.B. bei 15 bis 35°C, gibt dann das Säurehalogenid, vorzugsweise Phosphorpentachlorid, über eine Dosiervorrichtung zu. Man kann jedoch z.B. auch Phosphorpentachlorid in Phosphoroxychlorid suspendieren und dann in die Suspension der Sulfamidsäure II in einem der vorgenannten inerten Halogenkohlenwasserstoffe zulaufen lassen. Statt des Phosphorpentachlorids kann man auch vorteilhaft seine Ausgangsstoffe verwenden. Beispielsweise leitet man nach dem Verfahren der US—Patentschrift 1 906 440 in eine Lösung von Phosphortrichlorid in Phosphoroxychlorid die berechnete Menge Chlor ein und läßt diese Suspension dann in die Suspension der Sulfamidsäure II in einem der vorgenannten inerten Halogenkohlenwasserstoffe zulaufen. Nach der Verfahrensweise der gleichen Patentschrift kann man jedoch auch eine Mischung von gelbem Phosphor in Phosphoroxychlorid mit der berechneten Menge Chlor versetzen und dann in die Suspension der Sulfamidsäure einführen.

Bevorzugt ist folgende Arbeitsweise: Unter kräftigem Rühren gibt man das Säurehalogenid in eine Vorlage mit Ausgangsstoff II und Lösungsmittel. Die Zugabe dauert zweckmäßig von 10 bis 55 Minuten und erfolgt häufig bei Temperaturen von 20 bis 50°C, die Reaktion dann bei Temperaturen von mindestens 50°C.

Die nach dem Verfahren der Erfindung herstellbaren Verbindungen sind wertvolle Ausgangsstoffe für die Herstellung von Pflanzenschutzmitteln, Farbstoffen und Pharmazeutika. So kann man z.B. aus ihnen durch Umsetzung mit Anthranilsäure oder ihren Salzen, entsprechend dem Verfahren der deutschen Offenlegungsschrift 2 104 682, O-substituierte N-Hydroxy-o-sulfamidobenzoesäuren herstellen. Durch Cyclisierung dieser Stoffe, z.B. nach dem in der deutschen Offenlegungsschrift 2 105 687 beschriebenen Verfahren, gelangt man zu entsprechenden 2,1,3-Benzothiadiazin-4-

on-2,2-dioxiden, die für Pflanzenschutzmittel und Pharmazeutika verwendet werden können.

Durch Umsetzung der Endstoffe I mit substituierten Glykolsäureaniliden erhält man weitere Herbizide.

Die vorgenannten bevorzugten Endstoffe I sind bevorzugte Stoffe der vorgenannten Verwendung.

Die in den folgenden Beispielen aufgeführten Teile bedeuten Gewichtsteile.

Beispiel 1

208 Teile Phosphorpentachlorid werden unter Rühren bei 22°C in eine Suspension von 149 Teilen O-Methyl-N-hydroxysulfamidsaures Natriumsalz in 1 000 Teilen 1,2-Dichloräthan eingetragen. Anschließend wird das Reaktionsgemisch innerhalb 30 Minuten auf 75°C erwärmt und 2 Stunden bei 75°C bis 80°C gerührt. Das Reaktionsgemisch wird abfiltriert, das Filtrat im Vakuum von 1,2-Dichloräthan befreit, wobei man aus dem Rückstand durch fraktionierte Destillation 101 Teile (70% der Theorie) O-Methyl-N-hydroxysulfamidsäurechlorid mit Kp (0,2 mbar) 46 bis 48°C und $n_D^{25}$: 1,4527 erhält.

Beispiel 2

52 Teile Phosphorpentachlorid werden unter Rühren bei 0°C über eine Dosiervorrichtung in eine Suspension von 41 Teilen O-Äthyl-N-hydroxysulfamidsaures Natriumsalz in 45 Teilen Phosphoroxychlorid und 70 Teilen Benzol eingegeben. Anschließend wird das Reaktionsgemisch innerhalb 30 Minuten auf 80°C erwärmt und 2 Stunden bei 80 bis 82°C gerührt. Die fraktionierte Destillation des Reaktionsgemisches liefert 23,8 Teile (60% der Theorie) O-Äthyl-N-hydroxysulfamidsäurechlorid mit Kp (0,2 mbar) 58 bis 62°C und $n_D^{25}$: 1,4495.

Beispiel 3

208 Teile Phosphorpentachlorid, in 500 Teilen 1,2-Dichloräthan suspendiert, werden über eine Dosiervorrichtung bei 25°C zu einer Suspension von 233 Teilen O-n-Heptyl-N-hydroxysulfamidsaures Natriumsalz in 500 Teilen 1,2-Dichloräthan eingegeben. Das Reaktionsgemisch wird auf 75°C erwärmt, 3 Stunden bei 75°C bis 80°C gerührt, abgekühlt und filtriert. Nach fraktionierter Destillation unter Vakuum erhält man 121 Teile (53% der Theorie) O-n-Heptyl-N-hydroxysulfamidsäurechlorid vom Kp (0,5 mbar) 140 bis 145°C mit $n_D^{25}$: 1,4523

Beispiel 4

52 Teile Phosphorpentachlorid werden unter Rühren bei 25°C über eine Dosiervorrichtung in eine Suspension von 44 Teilen O-Isopropyl-N-hydroxysulfamidsaures Natriumsalz in 300 Teilen Chlorbenzol eingetragen. Das Reaktionsgemisch wird auf 75°C erwärmt, 2 Stunden bei 75°C bis 80°C gerührt, abgekühlt und filtriert. Nach fraktionierter Destillation unter Vakuum erhält man 25 Teile (58% der Theorie) O-Isopropyl-N-hydroxysulfamidsäurechlorid vom Kp (0,5 mbar) 138 bis 140°C mit $n_D^{25}$: 1,4516.

## Patentanspruch

Verfahren zur Herstellung von O-substituierten N-Hydroxysulfamidsäurehalogeniden der Formel

$$RO{-}\underset{\displaystyle H}{\underset{|}{N}}{-}SO_2X \qquad I,$$

in der R einen aliphatischen oder cycloaliphatischen Rest bedeutet und X ein Halogenatom bezeichnet, durch Umsetzung von Sulfamidsäuren mit einem Säurehalogenid, dadurch gekennzeichnet, daß man Sulfamidsäuren der Formel

$$RO{-}\underset{\displaystyle H}{\underset{|}{N}}{-}SO_3H \qquad II,$$

in der R die vorgenannte Bedeutung hat, oder ihrer sulfamidsauren Metallsalze mit einem Säurehalogenid der schwefligen Säure, Phosphorsäure, phosphorigen Säure, Kohlensäure oder Oxalsäure in einer Menge von 0,5 bis 1 Mol Säurehalogenid, bezogen auf 1 Mol Ausgangsstoff II, umsetzt.

**Revendication**

Procédé de préparation d'halogénures d'acides N-hydroxy-sulfamiques O-substitués de la formule

$$\begin{array}{c} RO\text{—}N\text{—}SO_2X \\ | \\ H \end{array} \qquad \text{I,}$$

dans laquelle R désigne un reste aliphatique ou cyclo-aliphatique et X un atome d'halogène, par réaction d'acides sulfamiques avec un halogénure d'acide, caractérisé en ce que l'on fait réagir des acides sulfamiques de la formule

$$\begin{array}{c} RO\text{—}N\text{—}SO_3H \\ | \\ H \end{array} \qquad \text{II,}$$

dans laquelle R possède les significations définies, ou leurs sels de métaux, avec un halogénure de l'acide sulfureux, de l'acide phosphorique, de l'acide phosphoreux, de l'acide carbonique ou de l'acide oxalique en une proportion de 0,5 à 1 mole d'halogénure pour 1 mole du composé de départ II.

**Claim**

A process for the preparation of an O-substituted N-hydroxy-sulfamic acid halide of the formula

$$\begin{array}{c} RO\text{—}N\text{—}SO_2X \\ | \\ H \end{array} \qquad \text{I}$$

where R is an aliphatic or cycloaliphatic radical and X is halogen, by reacting a sulfamic acid with an acid halide, characterized in that a sulfamic acid of the formula

$$\begin{array}{c} RO\text{—}N\text{—}SO_3H \\ | \\ H \end{array} \qquad \text{II}$$

where R has the above meaning, or a metal salt thereof, is reacted with an acid halide of sulfurous acid, phosphoric acid, phosphorous acid, carbonic acid or oxalic acid, using from 0.5 to 1 mole of acid halide per mole of starting material II.